# EUROPEAN PATENT SPECIFICATION

(11) **EP 2 943 427 B1**
(45) Date of publication and mention of the grant of the patent: **20.03.2019**
(21) Application number: 13871178.3
(22) Date of filing: 30.12.2013
(51) Int. Cl.: B65H 49/18, B65H 57/00, A61F 13/15, B65H 49/16

(54) **ELASTIC THREAD TRANSPORTING METHOD AND ASSEMBLY HAVING SIMPLIFIED PATH**
VERFAHREN UND ANORDNUNG ZUR FÖRDERUNG EINES ELASTISCHEN FADENS MIT EINEM VEREINFACHTEN WEG
ENSEMBLE ET PROCÉDÉ DE TRANSPORT DE FIL ÉLASTIQUE AYANT UN TRAJET SIMPLIFIÉ

(30) Priority: 14.01.2013 US 201361752106 P
(43) Date of publication of application: 18.11.2015
(73) Proprietor: Invista Technologies S.à.r.l., 9000 St. Gallen (CH)
(72) Inventor: BING-WO, Ronald, D., Waynesboro, VA 22980 (US)
(74) Representative: Carpmaels & Ransford LLP
(86) International application number: PCT/US2013/078232
(87) International publication number: WO 2014/109924

(56) References cited:
- EP-A1- 2 520 529
- EP-A2- 1 305 248
- JP-A- S57 133 201
- JP-A- 2000 160 460
- JP-A- 2011 136 816
- US-A- 4 417 935
- US-A- 5 236 539
- US-A- 5 389 173
- US-A1- 2002 023 706
- US-A1- 2003 057 232
- US-A1- 2005 126 689
- US-A1- 2006 225 465
- US-A1- 2012 071 852

## Description

### FIELD OF THE INVENTION:

The present invention relates generally to an elastic thread or yarn transport assembly for transporting thread from a thread creel to a production line. More particularly, the present invention is directed to a thread transporting assembly and method having a simplified path for feeding thread to the production line.

### BACKGROUND OF THE INVENTION:

In the manufacture of certain textiles, especially absorbent articles, such as disposable diapers, in order to improve the fitting of the diaper, a structure containing gathers from elastic threads are typically employed in the waistline portion and leg hole portions. These elastic threads are typically supplied from a thread supply device such as a thread creel to a production line in which the elastic thread is incorporated into the diaper. In such a supply device, the elastic thread is supplied from the creel and is transported over a tortuous path which includes a plurality of rollers, guides and the like for changing the direction of the thread and providing the proper tension. The rollers assure proper positioning of the threads with respect to the production line.

Supply apparatuses of this type are well known, as shown in U.S. Patent Publication No. 2011/0036487, U.S. Patent No. 7,878,447 and EP 2 520 529. Due to the complex path and guiding arrangement, is not uncommon for one or more thread lines to experience down time due to thread breakage. Also, as the number of threads employed increases, the chances of damage resulting in breakage also increases. When an individual elastic thread breaks it takes several minutes to reconnect the broken thread. During this period of time, the diaper production line must be stopped for the operator to gain access to the equipment so that the thread can be reconnected. These stoppages can greatly affect the overall efficiency of the diaper production line.

One of the significant factors in increasing the reconnection time is that thread breakage between the thread creel and the production line typically occurs at locations which are not normally accessible or difficult to access.

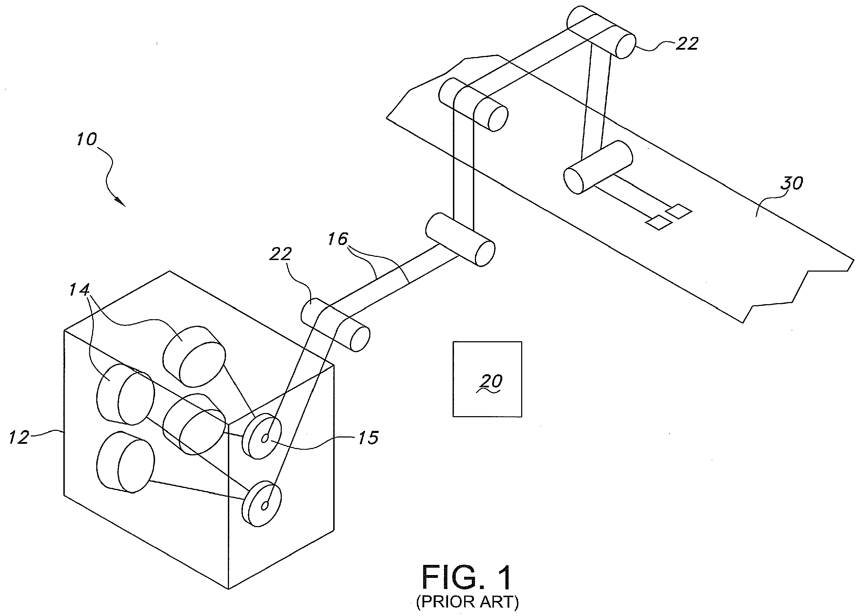

A schematic example of one such supply apparatus is shown in Figure 1. A supply apparatus 10 includes a thread creel 12 having a plurality of thread packages 14 fed to guides and feeders 15 from which threads 16 are unwound. In order to traverse obstacles or other equipment typically found on a manufacturing floor, schematically represented by 20, the threads are transported through a tortuous path. This path is facilitated by the use of a plurality of rollers 22 to assure proper placement of the threads in the production line 30. The rollers not only effect change of direction of threads but also may be tensioning rollers to apply the proper tension. Thread breakage between the creel 12 and the processing line 30 must be addressed by restringing the thread. However, as can be seen from Figure 1, due to the tortuous path which the thread must traverse and the number of obstacles which must be bypassed, it becomes difficult to quickly and efficiently reconnect the broken thread.

One technique to minimize thread breakage is to employ elastic or stretch films or stretch films or nettings which have a lower frequency of breakage. However, this solution is not advantageous in many situations.

It is, therefore, desirable to provide improved access to the threads in a production line to minimize the time it takes to reconnect broken thread.

### SUMMARY OF THE INVENTION:

The present invention is directed generally to an elastic tread transporting assembly for transporting thread to a production line. The assembly includes a thread creel for supplying multiple threads. The creel defines an unwinding location. A carrier is provided for transporting the threads to the production line along a carrier path. An attachment device attaches the threads to the carrier where the attachment device is placed in close proximity to the unwinding location using a minimal number of roller guides. The carrier path provides unrestricted access to the threads between the unwinding location and the production line.

In a preferred embodiment of the present invention, the attachment device includes a glue nozzle for gluing the threads to a carrier, where the glue nozzle is positioned in close proximity to the thread creel to facilitate quick repair and set-up.

In a method aspect of the present invention, a method of transporting multiple threads to a production line is provided. The method includes supplying the threads from a thread creel, said creel defining an unwinding location. The threads are then attached to a carrier at a location in close proximity to the thread creel using a minimal number of roller guides. The threads are transported on the carrier to the production line along a carrier path, said carrier path providing unrestricted access to said threads between said unwinding location and said production line.

### BRIEF DESCRIPTION OF THE DRAWINGS:

Figure 1 is a schematic representation of a transporting assembly of the prior art.
Figure 2 is a schematic representation of a transporting assembly arranged in accordance with the present invention.
Figure 3 is a schematic representation of an alternate embodiment of a transporting assembly, which does not form part of the present invention.

### DETAILED DESCRIPTION OF THE PREFERRED EMBODIMENTS:

The present invention provides a transporting assembly which transports a plurality of threads to a production line by providing a simplified and direct path between the supply of the threads and the insertion of the threads into the production line.

In the preferred embodiment of the present invention, the production line is a line used for the manufacture of disposable diapers and the threads are elastic threads which are useful for forming the waist bands, belly bands, and leg holes gathers for such disposable diapers. While the preferred embodiment of the present invention is useful in forming disposable diapers using elastic threads, the present invention is not limited thereto.

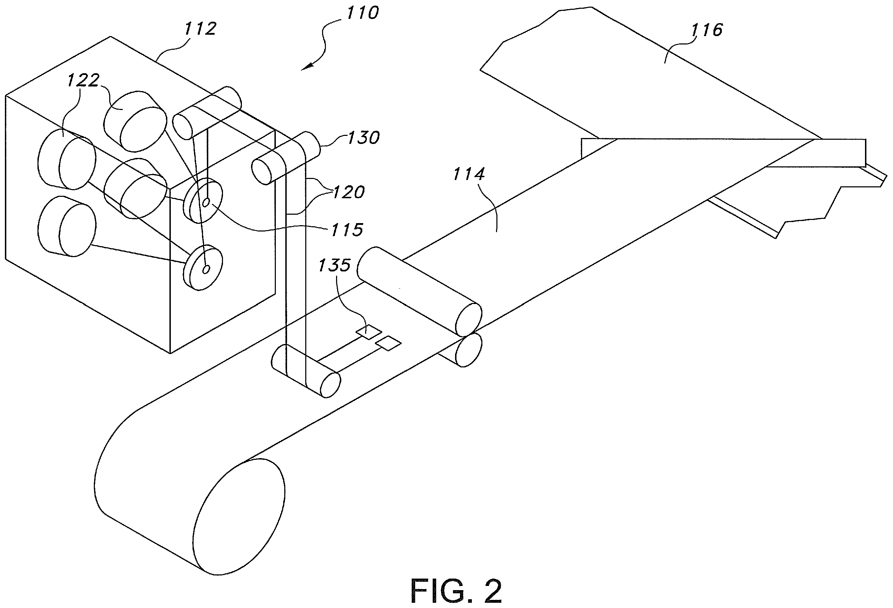

Referring now to Figure 2, one embodiment of the transporting assembly of the present invention is shown. Transporting assembly 110 includes a thread creel 112, a carrier 114, and a production line 116 where the threads are incorporated into the product, in this case, disposable diapers.

Threads 120 are unwound from thread packages 122 using reels and feeders 115 contained within the thread creel 112. The threads 120 are transported to a carrier 114 which is typically a non-woven textile material which supports the threads and is incorporated into the production line 116 for formation of the diaper. While two threads are shown for clarity, it is understood that multiple threads are typically employed. The carrier web 114 is located at a position in close proximity to the thread creel 112 so that the threads 120 may be transferred to the carrier web 114 with minimal distance and change of direction therebetween. Rollers 130 may be employed in this short distance to accurately position and tension the threads. Upon bringing together the threads 120 and the carrier web 114, the threads 120 may be glued to the carrier web by glue nozzles 135 of conventional adhesive attachment equipment well-known in the art located at the beginning of the path of the carrier web. Once the threads 120 are affixed or glued to the carrier web 14, the threads and the carrier web are transported to the production line.

The distance between the thread creel 112 and the carrier web 14 is kept to a minimum and is at an accessible point so that if thread breakage occurs between the thread creel and the carrier web it can be easily repaired. After the threads are attached to the carrier web, there is less likelihood of thread breakage and, therefore, the carrier web can transport the threads to the production line 116.

As mentioned above, the method and apparatus of the present invention is useful for high thread count applications. In such situations, it is often necessary to further process the carrier web with the threads attached thereto so as to facilitate incorporation into the production line 116. The carrier web could be further processed by slitting or folding the carrier web to separate the slitted or folded carrier web to facilitate introduction into the production line so as to optimize use within different equipment layouts.

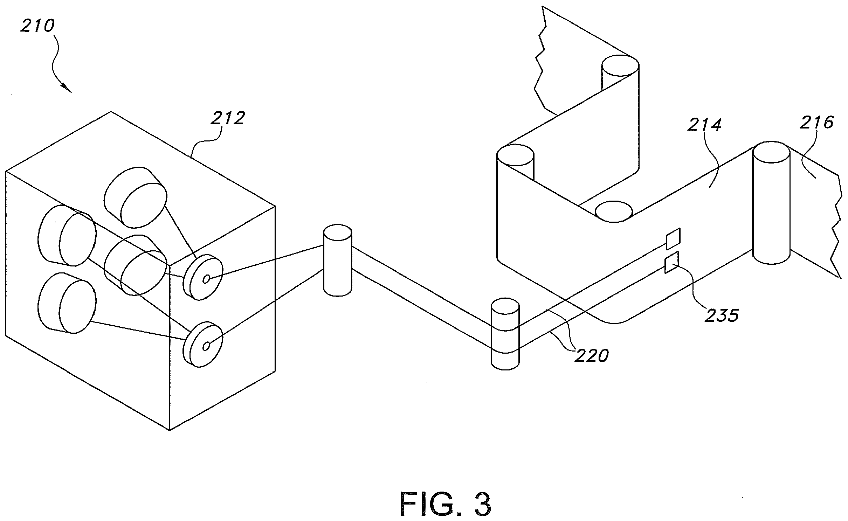

Referring now to Figure 3, an alternative embodiment of a transporting assembly, which does not form part of the present invention, is shown. Transporting assembly 210 includes a thread creel 212 and a production line 216. The production line itself has an offset configuration so as to provide a carrier web portion 214 which is in close proximity to thread creel 212. While in Figure 3, the carrier web is shown arranged vertically for clarity, more typically, the carrier web is oriented horizontally with respect to the thread creel.

In the embodiment shown in figure 3, the threads 220 from creel 212 are transported to the carrier portion 214 of processing line 216. Again, glue nozzles 235 attach the threads 220 to the carrier 214. As with the above described embodiment, the threads 220 makes minimal change in direction between the creel 212 and the carrier portion 214. This minimizes thread breakage and any thread breakage which occurs can be easily accessed.

Various changes to the foregoing described and shown structures would now be evident to those skilled in the art. Accordingly, the particularly disclosed scope of the invention is set forth in the following claims.

## Claims

1. An elastic thread transporting assembly for transporting thread to a production line comprising:
a thread creel for supplying multiple threads, said creel defining an unwinding location;
a carrier for transporting said threads to said production line along a carrier path;
an attachment device for attaching said threads to said carrier, said attachment device being placed in close proximity to said unwinding location;
said carrier path providing unrestricted access to said threads between said unwinding location and said production line.

2. An assembly of claim 1 wherein said attachment device includes a glue nozzle for gluing said threads to said carrier.

3. An assembly of claim 2 wherein said glue nozzle is located adjacent to said thread creel.

4. An assembly of claim 1 wherein said carrier is non-woven.

5. An assembly of claim 1 wherein said carrier supplies said threads to said production line in line with movement of said production line.

6. An assembly of claim 1 wherein said carrier supplies said threads to said production line to offset movement of said production line.

7. A method of transporting multiple threads to a production line comprising the steps of:
supplying said threads from a thread creel, said creel defining an unwinding location;
attaching said threads to a carrier at a location in close proximity to said unwinding location; and
transporting said threads on said carrier to said production line along a carrier path; said carrier path providing unrestricted access to said threads between said unwinding location and said production line.

8. A method of claim 7 wherein said supply step includes:
unwinding said thread from said creel.

9. A method of claim 7 wherein said attaching step includes:
gluing said thread to said carrier.

10. A method of claim 7 wherein said carrier if formed of non-woven material.

11. A method of claim 9 wherein said gluing step includes:
providing a glue nozzle positioned adjacent said creel.

## Patentansprüche

1. Anordnung zum Transportieren eines elastischen Fadens zum Transportieren eines Fadens zu einer Fertigungsstrecke, Folgendes umfassend:
einen Fadenständer zum Liefern mehrerer Fäden, wobei der Ständer eine Abwickelstelle definiert;
einen Träger zum Transportieren der Fäden zur Fertigungsstrecke entlang eines Trägerweges;
eine Befestigungsvorrichtung zum befestigen der Fäden an dem Träger, wobei die Befestigungsvorrichtung in der Nähe der Abwickelstelle aufgestellt ist;
wobei der Trägerweg zwischen der Abwickelstelle und der Fertigungsstrecke uneingeschränkten Zugriff auf die Fäden bereitstellt.

2. Anordnung nach Anspruch 1, wobei die Befestigungsvorrichtung eine Klebstoffdüse enthält, um die Fäden auf den Träger zu kleben.

3. Anordnung nach Anspruch 2, wobei die Klebstoffdüse an den Fadenständer angrenzt.

4. Anordnung nach Anspruch 1, wobei der Träger ein Vlies ist.

5. Anordnung nach Anspruch 1, wobei der Träger der Fertigungsstrecke die Fäden in Übereinstimmung mit der Bewegung der Fertigungsstrecke liefert.

6. Anordnung nach Anspruch 1, wobei der Träger der Fertigungsstrecke die Fäden versetzt zu der Bewegung der Fertigungsstrecke liefert.

7. Verfahren zum Transportieren mehrerer Fäden zu einer Fertigungsstrecke, die folgenden Schritte umfassend:
Liefern der Fäden von einem Fadenständer, wobei der Ständer eine Abwickelstelle definiert;
Befestigen der Fäden an einem Träger an einer Stelle in der Nähe der Abwickelstelle; und
Transportieren der Fäden auf dem Träger zu der Fertigungsstrecke entlang eines Trägerweges; wobei der Trägerweg zwischen der Abwickelstelle und der Fertigungsstrecke uneingeschränkten Zugriff auf die Fäden bereitstellt.

8. Verfahren nach Anspruch 7, wobei der Lieferschritt Folgendes enthält:
Abwickeln des Fadens von dem Fadenständer.

9. Verfahren nach Anspruch 7, wobei der Befestigungsschritt Folgendes umfasst:
Kleben des Fadens auf den Träger.

10. Verfahren nach Anspruch 7, wobei der Träger aus Vlies gebildet ist.

11. Verfahren nach Anspruch 9, wobei der Klebeschritt Folgendes enthält:
Bereitstellen einer Klebstoffdüse, die in an den Ständer angrenzt.

## Revendications

1. Ensemble de transport de fil élastique pour transporter un fil jusqu'à une ligne de production, comprenant :
une bobine de fil pour fournir de multiples fils, ladite bobine définissant un point de débobinage ;
un support pour transporter lesdits fils jusqu'à ladite ligne de production le long d'un chemin de support ;
un dispositif de fixation pour attacher lesdits fils audit support, ledit dispositif de fixation étant placé dans le voisinage immédiat dudit point de débobinage ;
ledit chemin de support offrant un accès illimité auxdits fils entre ledit point de débobinage et ladite ligne de production.

2. Ensemble selon la revendication 1, dans lequel ledit dispositif de fixation comprend une buse à colle pour coller lesdits fils audit support.

3. Ensemble selon la revendication 2, dans lequel ladite buse à colle est située à proximité de ladite bobine de fil.

4. Ensemble selon la revendication 1, dans lequel ledit support est non tissé.

5. Ensemble selon la revendication 1, dans lequel ledit support fournit lesdits fils à ladite ligne de production en ligne avec le déplacement de ladite ligne de production.

6. Ensemble selon la revendication 1, dans lequel ledit support fournit lesdits fils à ladite ligne de production de manière à décaler le déplacement de ladite ligne de production.

7. Procédé de transport de multiples fils jusqu'à une ligne de production, comprenant les étapes suivantes :
fournir lesdits fils à partir d'une bobine de fil, ladite bobine définissant un point de débobinage ;
attacher lesdits fils à un support en un point qui est situé dans le voisinage immédiat dudit point de débobinage ; et
transporter lesdits fils sur ledit support jusqu'à ladite ligne de production le long d'un chemin de support, ledit chemin de support offrant un accès illimité auxdits fils entre ledit point de débobinage et ladite ligne de production.

8. Procédé selon la revendication 7, dans lequel ladite étape de fourniture comprend le débobinage dudit fil à partir de ladite bobine.

9. Procédé selon la revendication 7, dans lequel ladite étape de fixation comprend le collage dudit fil audit support.

10. Procédé selon la revendication 7, dans lequel ledit support est constitué d'un matériau non tissé.

11. Procédé selon la revendication 9, dans lequel ladite étape de collage comprend la disposition d'une buse à colle positionnée à proximité de ladite bobine.
